# EUROPÄISCHE PATENTSCHRIFT

(11) **EP 0 764 642 B1**
(45) Veröffentlichungstag und Bekanntmachung des Hinweises auf die Patenterteilung: **04.12.2002**
(21) Anmeldenummer: 96114442.5
(22) Anmeldetag: 10.09.1996
(51) Int. Cl.: C07D 257/04

(54) **Verfahren zur Herstellung von 1-Aryl-4-carbamoyl-tetrazolinonen**
Process for the production of 1-Aryl-4-carbamoyl-tetrazolinones
Procédé pour la préparation de 1-aryl-4-carbamoyl-tétrazolinones

(30) Priorität: 22.09.1995 DE 19535242
(43) Veröffentlichungstag der Anmeldung: 26.03.1997
(73) Patentinhaber: Bayer CropScience AG, 40789 Monheim (DE)
(72) Erfinder: Lantzsch, Reinhard, Dr., 42115 Wuppertal (DE); Stelzer, Uwe, Dr., 51399 Burscheid (DE); Casser, Carl, Dr., 13585 Berlin (DE)

(56) Entgegenhaltungen:
- EP-A- 0 146 279
- EP-A- 0 572 855
- EP-A- 0 578 090
- EP-A- 0 646 577
- EP-A- 0 726 259
- US-A- 4 044 018
- J.ORG.CHEM., Bd. 45, 1980, WASHINGTON, Seiten 5130-5136, XP002023244 TSUGE,O. ET AL.: "Reactions of Trimethylsilyl Azide with Heterocumulenes "

## Beschreibung

Die Erfindung betrifft ein neues Verfahren und neue Zwischenprodukte zur Herstellung von 1-Aryl-4-carbamoyl-tetrazolinonen, welche als herbizid wirksame Verbindungen bekannt sind.

Es ist bekannt, dass man substituierte Carbamoyltetrazolinone erhält, wenn man entsprechende Tetrazolinone mit geeigneten Carbamidsäurederivaten umsetzt (vgl. EP-A 146 279, EP-A 202 929, EP-A 578 090, EP-A 572 855, EP-A 726 259, EP-A 646 577 und EP-A 612 735). Bei dieser Herstellungsweise wird neben der erwünschten N-Carbamoylierung immer auch eine (unerwünschte) O-Carbamoylierung beobachtet (zur Acylierung von Tetrazolinonen vgl. auch Z. Chemie 13 (1973), 429-430). Man erhält also in vielen Fällen mehr oder weniger stark verunreinigte Produkte. Es ist auch bekannt, dass man 1-Aryl-4-carbamoyl-tetrazolinone erhält, wenn man Arylisocyanate mit Trimethylsilylaziden umsetzt (J. Org. Chem. 45, (1980), 5130-5136).

Weiter ist bekannt, dass man 1-Alkyl-4-chlorcarbonyl-tetrazolinone erhält, wenn man 1-Alkyl-tetrazolinone mit Phosgen in Gegenwart eines tertiären Amins umsetzt (vgl. US 4 830 661). Die Phosgenierung zu den 1-Alkyl-4-carbamoyl-tetrazolinonen wird bei dieser Herstellungsweise in Gegenwart einer Hilfsbase durchgeführt. 1-Aryl-4-chlorcarbonyl-tetrazolinone sind jedoch noch nicht aus der Literatur bekannt und können nach dem bekannten Verfahren auch nicht in brauchbarer Ausbeute und Qualität erhalten werden.

Die Herstellung von 2-Carbamoyl-isoxazolonen durch Phosgenierung von Isoxazolonen mit Phosgen und anschließende Umsetzung des Reaktionsproduktes mit Aminen ist ebenfalls bekannt (U.S. 4,044,018). Generell ist die Phosgenierung von sekundären Aminen zu den entsprechenden Carbaminsäuren bekannt (DE-A 22 06 365; Houben-Weyl, Methoden der Organischen Chemie, Band E4, (1983), Verlag G. Thieme, Seite 335-8).

Es wurde nun gefunden, dass man 1-Aryl-4-carbamoyl-tetrazolinone der allgemeinen Formel (I) 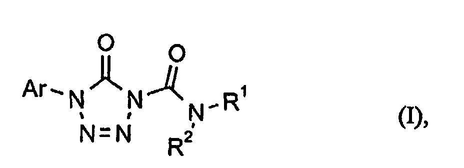 in welcher
- Ar: für gegebenenfalls durch Carboxy, Cyano, Carbamoyl, Nitro, Amino, Hydroxy, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl, C₁-C₄-Alkylcarbonylamino, C₁-C₄-Alkoxy-carbonyl, Di-(C₁-C₄-alkylamino)-carbonyl, C₁-C₄-Alkylendioxy, Phenyl oder Phenoxy (welche jeweils gegebenenfalls durch Fluor und/oder Chlor substituiert sind) substituiertes Phenyl oder Naphthyl steht,
- R¹: für jeweils gegebenenfalls durch Cyano oder Halogen substituiertes Alkyl, Alkenyl oder Alkinyl mit jeweils 1 bis 6 Kohlenstoffatomen steht und
- R²: für gegebenenfalls durch Cyano oder Halogen substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 2 Kohlenstoffatomen im Alkylteil, oder für jeweils gegebenenfalls durch Cyano, Nitro, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl oder C₁-C₄-Alkoxy-carbonyl (welche jeweils gegebenenfalls durch Fluor und/oder Chlor substituiert sind) substituiertes Phenyl oder Phenyl-C₁-C₂-alkyl steht,
oder zusammen mit R¹ für Alkandiyl mit 2 bis 6 Kohlenstoffatomen steht,
in sehr guten Ausbeuten und in hoher Reinheit erhält, wenn man
1-Aryl-tetrazolinone der allgemeinen Formel (II) 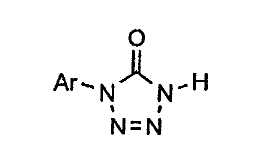 in welcher
- Ar: die oben angegebene Bedeutung hat,
mit Phosgen in Gegenwart eines Verdünnungsmittels und in Abwesenheit einer Hilfsbase bei Temperaturen zwischen 0°C und 150°C umsetzt ("erste Verfahrensstufe") und die hierbei gebildeten 1-Aryl-4-chlorcarbonyl-tetrazolinone der allgemeinen Formel (III) 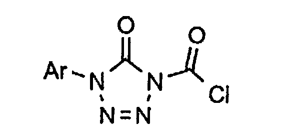 in welcher
- Ar: die oben angegebene Bedeutung hat,
- gegebenenfalls nach Zwischenisolierung oder ohne Zwischenisolierung -
mit Aminen der allgemeinen Formel (IV) 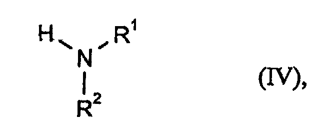 in welcher
- R¹ und R²: die oben angegebene Bedeutung haben,
in Gegenwart eines Verdünnungsmittels und gegebenenfalls in Gegenwart einer weiteren basischen Verbindung bei Temperaturen zwischen -20°C und +100°C umsetzt ("zweite Verfahrensstufe").

Überraschenderweise können nach dem erfindungsgemäßen Verfahren 1-Aryl-4-carbamoyl-tetrazolinone der allgemeinen Formel (I) auf einfachere, ökonomisch günstigere Weise sowie in höheren Ausbeuten und in besserer Qualität als nach den bekannten Methoden hergestellt werden.

Das exfindungsgemäße Verfahren stellt somit eine wertvolle Bereicherung des Standes der Technik dar.

Das erfindungsgemäße Verfahren betrifft insbesondere die Herstellung von Verbindungen der Formel (I), in welcher
- Ar: für gegebenenfalls durch Cyano, Fluor, Chlor, Brom, durch Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Dimethylaminosulfonyl, Diethylaminosulfonyl, Methylcarbonyl, Ethylcarbonyl, n- oder i-Propylcarbonyl, Methoxycarbonyl, Ethoxycarbonyl, n- oder i-Propoxycarbonyl, Dimethylaminocarbonyl, Diethylaminocarbonyl, Methylendioxy oder Ethylendioxy (welche jeweils gegebenenfalls durch Fluor und/oder Chlor substituiert sind) substituiertes Phenyl steht,
- R¹: für jeweils gegebenenfalls durch Cyano, Fluor oder Chlor substituiertes Methyl, Ethyl, n- oder i-Propyl, n-, i- oder s-Butyl, Propenyl, Butenyl, Propinyl oder Butinyl steht und
- R²: für jeweils gegebenenfalls durch Cyano, Fluor oder Chlor substituiertes Methyl, Ethyl, n- oder i-Propyl, n-, i- oder s-Butyl, Propenyl, Butenyl, Propinyl oder Butinyl, für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Cyano, Fluor, Chlor, Methyl, Ethyl, Methoxy oder Ethoxy substituiertes Phenyl, Benzyl oder Phenylethyl steht,
oder zusammen mit R¹ für Butan-1,4-diyl (Tetramethylen) oder Pentan-1,5-diyl (Pentamethylen) steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise in der ersten Stufe 1-(2-Fluor-phenyl)-1,4-dihydro-5Htetrazol-5-on und Phosgen sowie in der zweiten Stufe Diethylamin als Ausgangsstoffe, so kann der Reaktionsablauf beim erfindungsgemäßen Verfahren durch das folgende Formelschema skizziert werden:

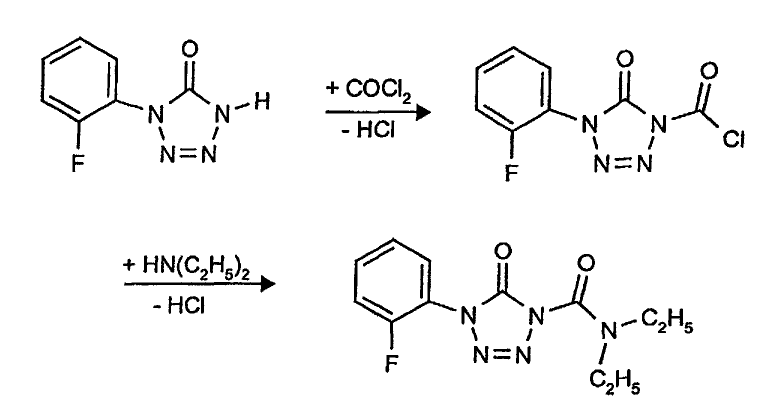

Die beim erfindungsgemäßen Verfahren zur Herstellung der Verbindungen der Formel (I) als Ausgangsstoffe zu verwendenden 1-Aryl-tetrazolinone sind durch die Formel (II) allgemein definiert. In der Formel (II) hat Ar insbesondere diejenige Bedeutung, die bereits oben bei der Beschreibung der erfindungsgemäß herzustellenden Verbindungen der Formel (I) als insbesondere bevorzugt für Ar angegeben wurde.

Die Ausgangsstoffe der Formel (II) sind bekannt und/oder können nach an sich bekannten Verfahren hergestellt werden (vgl. J. Am. Chem. Soc. 81 (1959), 3076-3079; J. Org. Chem. 45 (1980), 5130-5136; EP-A 146279; EP-A 572855; EP-A 578090).

Die bei der ersten Stufe des erfindungsgemäßen Verfahrens zur Herstellung der Verbindungen der Formel (I) gebildeten 1-Aryl-4-chlorcarbonyl-tetrazolinone sind durch die Formel (III) allgemein definiert. In der Formel (III) hat Ar bzw. insbesondere diejenige Bedeutung, die bereits oben bei der Beschreibung der erfindungsgemäß herzustellenden Verbindungen der Formel (I) vorzugsweise bzw. als insbesondere bevorzugt für Ar angegeben wurde.

Die 1-Aryl-4-chlorcarbonyl-tetrazolinone der allgemeinen Formel (III) sind noch nicht aus der Literatur bekannt; sie sind als neue Stoffe auch Gegenstand der vorliegenden Anmeldung.

Die beim erfindungsgemäßen Verfahren zur Herstellung der Verbindungen der Formel (I) - in der zweiten Stufe - weiter als Ausgangsstoffe zu verwendenden Amine sind durch die Formel (IV) allgemein definiert. In der Formel (IV) haben R¹ und R² vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R¹ und R² angegeben wurde.

Die Ausgangsstoffe der Formel (IV) sind bekannte Synthesechemikalien.

Das erfindungsgemäße Verfahren zur Herstellung der Verbindungen der Formel (I) wird in Gegenwart eines Verdünnungsmittels durchgeführt. Als Verdünnungsmittel kommen für die erste Stufe vorzugsweise gegenüber Phosgen inerte organische Lösungsmittel in Betracht. Hierzu gehören insbesondere aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethyl-t-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon; Nitrile, wie beispielsweise Acetonitril, Propionitril, Butyronitril oder Benzonitril; Ester, wie beispielsweise Essigsäure-methylester, -ethyl ester, -n- oder -i-propylester, -n-, -i- oder -s-butylester; Sulfoxide, wie beispielsweise Dimethylsulfoxid.

Als besonders bevorzugte Verdünnungsmittel für die erste Stufe seien Toluol, Xylol, Dichlorethan und Chlorbenzol genannt.

Für die zweite Verfahrensstufe können grundsätzlich die gleichen Verdünnungsmittel wie bei Durchführung der ersten Verfahrensstufe verwendet werden. Als besonders bevorzugte Verdünnungsmittel sind Tetrahydrofuran und Chlorbenzol (letzteres insbesondere bei Arbeiten im "Eintopfverfahren") zu nennen.

Das erfindungsgemäße Verfahren zur Herstellung der Verbindungen der Formel (I) wird in der zweiten Stufe gegebenenfalls in Gegenwart einer weiteren basischen Verbindung - neben dem Amin der Formel (IV) - durchgeführt. Als basische Verbindungen kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n-oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8-Diazabicyclo[5,4,0]-undec-7-en (DBU).

In einer bevorzugten Ausführungsform des erfindungsgemäßen Verfahrens wird das Amin der Formel (IV) in einem entsprechenden Überschuß eingesetzt, somit als Base verwendet und es wird auf den Einsatz einer weiteren basischen Verbindung verzichtet.

Die Reaktionstemperaturen können bei der Durchführung des erfindungsgemäßen Verfahrens in einem größeren Bereich variiert werden. Im allgemeinen arbeitet man in der ersten Stufe bei Temperaturen zwischen 0°C und 150°C, vorzugsweise zwischen 50°C und 120°C, in der zweiten Stufe bei Temperaturen zwischen -20°C und +100°C, vorzugsweise zwischen 0°C und 80°C.

Das erfindungsgemäße Verfahren wird in beiden Stufen im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens setzt man je Mol Ausgangsverbindung der Formel (II) im allgemeinen zwischen 1 und 10 Mol, vorzugsweise zwischen 2 und 5 Mol Phosgen (erste Stufe) und zwischen 1 und 4 Mol, vorzugsweise zwischen 2 und 3 Mol Amin der Formel (IV) (zweite Stufe) ein.

In einer bevorzugten Ausführungsform der ersten Stufe des erfindungsgemäßen Verfahrens wird das Phosgen in einem Verdünnungsmittel vorgelegt und das 4-Aryl-tetrazolinon der Formel (II) wird langsam dazu gegeben. Die Reaktionsmischung wird dann - vorzugsweise unter Einleiten von Phosgen - auf die jeweils erforderliche Reaktionstemperatur aufgeheizt und - vorzugsweise unter weiterem Einleiten von Phosgen - bei dieser Temperatur gehalten, bis die Umsetzung zu den Verbindungen der Formel (III) beendet ist. Überschüssiges Phosgen wird dann - vorzugsweise durch Einleiten von Stickstoff - weitgehend entfernt. Das Zwischenprodukt der Formel (III) kann durch sorgfältiges Abdestillieren des Verdünnungsmittels unter vermindertem Druck isoliert werden.

In einer weiteren bevorzugten Ausführungsform des erfindungsgemäßen Verfahrens ("Eintopfverfahren") wird das Zwischenprodukt der Formel (III) nicht isoliert und ein Amin der Formel (IV) wird direkt zur Reaktionslösung, die nach Durchführung der ersten Stufe (nach der Entfernung des überschüssigen Phosgens) vorliegt, gegeben. Die Mischung wird dann bis zum Ende der Umsetzung gerührt und nach üblichen Methoden aufgearbeitet. Beispielsweise wird - gegebenenfalls nach Einengen und Aufnehmen in einem mit Wasser praktisch nicht mischbaren organischen Lösungsmittel, wie z.B. Methylenchlorid - mit Wasser gewaschen, getrocknet, filtriert und unter vermindertem Druck sorgfältig eingeengt, wobei das Produkt der Formel (I) als Rückstand verbleibt.

Die erfindungsgemäß herstellbaren 1-Aryl-4-carbamoyl-tetrazolinone der Formel (I) können als Herbizide zur Bekämpfung unerwünschten Pflanzenwuchses verwendet werden (vgl. EP-A 146279, EP-A 202929, EP-A 578090 und EP-A 612735).

### Herstellungsbeispiele:

### Beispiel 1

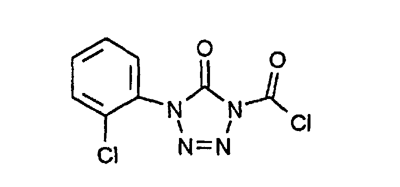

### (1. Stufe)

In eine Lösung von 7,4 g (75 mMol) Phosgen in 50 ml Toluol werden 4,9 g (25 mMol) 1-(2-Chlor-phenyl)-1,4-dihydro-5H-tetrazol-5-on portionsweise eingetragen. Unter weiterem Einleiten von Phosgen wird die Mischung auf 60°C aufgeheizt. Dann wird die Reaktionsmischung innerhalb von ca. 3 Stunden unter weiterem schwachen Einleiten von Phosgen auf Rückflußtemperatur gebracht und dann wird noch ca. 60 Minuten weiter Phosgen eingeleitet. Anschließend wird überschüssiges Phosgen mit Stickstoff ausgeblasen und die verbleibende Lösung im Wasserstrahlvakuum sorgfältig eingeengt.

Man erhält 6,4 g (99 % der Theorie) 1-(2-Chlor-phenyl)-4-chlorcarbonyl-1,4-dihydro-5H-tetrazol-5-on als weißen kristallinen Rückstand vom Schmelzpunkt 188°C (unter Zersetzung).

Das IR-Spektrum des Produktes zeigt eine für Carbonylgruppen charakteristische Absorption bei 1810 cm⁻¹.

### Beispiel 2

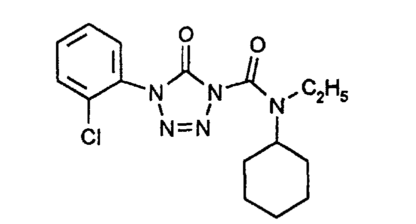

### (2. Stufe)

6,4 g (25 mMol) 1-(2-Chlor-phenyl)-4-chlorcarbonyl-1,4-dihydro-5H-tetrazol-5-on werden in 50 ml Tetrahydrofuran gelöst und eine Lösung von 6,9 g (54 mMol) N-Ethyl-cyclohexylamin in 30 ml Tetrahydrofuran wird unter Rühren tropfenweise dazu gegeben. Die Reaktionsmischung wird 2 Stunden bei ca. 20°C gerührt und dann im Wasserstrahlvakuum eingeengt. Der Rückstand wird in Methylenchlorid aufgenommen, dreimal mit Wasser gewaschen, mit Natriumsulfat getrocknet und filtriert. Vom Filtrat wird das Lösungsmittel im Wasserstrahlvakuum sorgfältig abdestilliert.

Man erhält 7,9 g (91 % der Theorie) 1-(2-Chlor-phenyl)-4-(N-cyclohexyl-N-ethylaminocarbonyl)-1,4-dihydro-5H-tetrazol-5-on als weißen kristallinen Rückstand vom Schmelzpunkt 78°C.

### Beispiel 3

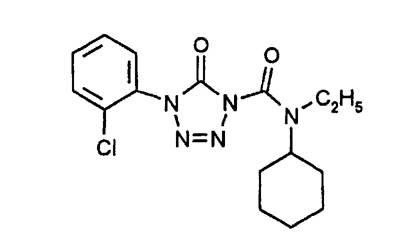

### (1. + 2. Stufe)

9,9 g Phosgen (100 mmol) werden in 200 ml Chlorbenzol vorgelegt und 9,8 g (50 mMol) 1-(2-Chlor-phenyl)-1,4-dihydro-5H-tetrazol-5-on werden portionsweise dazu gegeben. Die Mischung wird eine Stunde bei ca. 20°C gerührt und dann langsam unter weiterem Einleiten von Phosgen auf ca. 100°C aufgeheizt, wobei eine klare Lösung entsteht. Das Einleiten von Phosgen wird noch eine weitere Stunde fortgesetzt und die Mischung wird dann noch eine weitere Stunde bei ca. 100°C gerührt. Überschüssiges Phosgen und Chlorwasserstoff werden mit Stickstoff ausgeblasen. Anschließend wird - nach Abkühlen auf ca. 20°C - eine Lösung von 12,7 g (100 mMol) N-Ethyl-cyclohexylamin in 30 ml Chlorbenzol tropfenweise unter Rühren zum Reaktionsgemisch gegeben und das Rühren wird noch ca. eine Stunde fortgesetzt. Dann wird dreimal mit Wasser gewaschen, mit Natriumsulfat getrocknet und filtriert. Vom Filtrat wird das Lösungsmittel im Wasserstrahlvakuum sorgfältig abdestilliert.

Man erhält 15,9 g (91 % der Theorie über beide Stufen) 1-(2-Chlor-phenyl)-4-(Ncyclohexyl-N-ethyl-aminocarbonyl)-1,4-dihydro-5H-tetrazol-5-on als weißen kristallinen Rückstand vom Schmelzpunkt 77°C.

### Vergleichsbeispiel (analog US 4830661, Example 1)

Eine 23,3%ige Lösung von Phosgen in Toluol (85 g, 0,2 Mol) wird mit 100 ml Toluol verdünnt und auf 10°C abgekühlt. Eine Lösung von 41,4 g (0,16 Mol) 1-(2-Chlor-phenyl)-1,4-dihydro-5H-tetrazol-5-on, 17 g (0,168 Mol) Triethylamin und 0,2 g (1,6 mMol) 4-Dimethylamino-pyridin in 70 ml Toluol wird innerhalb von ca. 10 Minuten tropfenweise dazu gegeben, wobei die Temperatur der Reaktionsmischung unterhalb von 28°C gehalten wird. Die Mischung wird ca. 30 Minuten bei ca. 20°C gerührt und dann filtriert. Das Filtrat wird im Wasserstrahlvakuum eingeengt, der Rückstand in Methylenchlorid aufgenommen dreimal mit Wasser gewaschen, mit Natriumsulfat getrocknet und filtriert. Vom Filtrat wird im Wasserstrahlvakuum das Lösungsmittel sorgfältig abdestilliert.

Man erhält 45,7 g eines dunklen, zähen Öls folgender Zusammensetzung:

| | |
|---|---|
| 44,2 % | 1-(2-Chlor-phenyl)-4-(N-cyclohexyl-N-ethyl-aminocarbonyl)-1,4-dihydro-5H-tetrazol-5-on, |
| 44,2 % | 1-(2-Chlor-phenyl)-1,4-dihydro-5H-tetrazol-5-on und |
| 8 % | 1-(2-Chlor-phenyl)-3-cyclohexyl-3-ethyl-harnstoff. |

## Patentansprüche

1. Verfahren zur Herstellung von 1-Aryl-4-carbamoyltetrazolinonen der allgemeinen Formel (I) 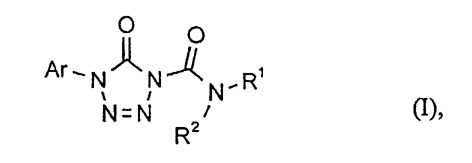 in welcher
Ar für gegebenenfalls durch Carboxy, Cyano, Carbamoyl, Nitro, Amino, Hydroxy, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl, C₁-C₄-Alkyl-carbonylamino, C₁-C₄-Alkoxycarbonyl, Di-(C₁-C₄-alkylamino)-carbonyl, C₁-C₄-Alkylendioxy, Phenyl oder Phenoxy (welche jeweils gegebenenfalls durch Fluor und/oder Chlor substituiert sind) substituiertes Phenyl oder Naphthyl steht,
R¹ für jeweils gegebenenfalls durch Cyano oder Halogen substituiertes Alkyl, Alkenyl oder Alkinyl mit jeweils 1 bis 6 Kohlenstoffatomen steht und
R² für gegebenenfalls durch Cyano oder Halogen substituiertes Alkyl mit 1 bis 6 Kohlenstoffatomen, für jeweils gegebenenfalls durch Cyano oder Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen im Cycloalkylteil und gegebenenfalls 1 bis 2 Kohlenstoffatomen im Alkylteil, oder für jeweils gegebenenfalls durch Cyano, Nitro, Halogen, durch C₁-C₄-Alkyl, C₁-C₄-Alkoxy, C₁-C₄-Alkylthio, C₁-C₄-Alkylsulfinyl, C₁-C₄-Alkylsulfonyl, Di-(C₁-C₄-alkyl)amino, C₁-C₄-Alkylsulfonylamino, Di-(C₁-C₄-alkyl)aminosulfonyl, C₁-C₄-Alkyl-carbonyl oder C₁-C₄-Alkoxy-carbonyl (welche jeweils gegebenenfalls durch Fluor und/oder Chlor substituiert sind) substituiertes Phenyl oder Phenyl-C₁-C₂-alkyl steht,
oder zusammen mit R¹ für Alkandiyl mit 2 bis 6 Kohlenstoffatomen steht,
**dadurch gekennzeichnet, daß** man 1-Aryl-tetrazolinone der allgemeinen Formel (II) 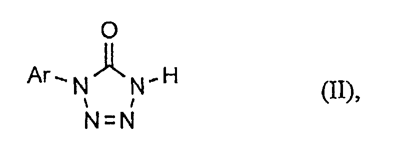 in welcher
Ar die oben angegebene Bedeutung hat,
mit Phosgen in Gegenwart eines Verdünnungsmittels und in Abwesenheit einer Hilfsbase bei Temperaturen zwischen 0°C und 150°C umsetzt ("erste Verfahrensstufe") und die hierbei gebildeten 1-Aryl-4-chlorcarbonyl-tetrazolinone der allgemeinen Formel (III) 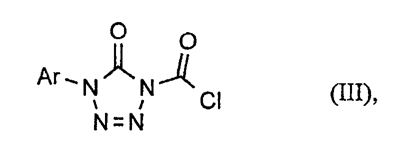 in welcher
Ar die oben angegebene Bedeutung hat,
- gegebenenfalls nach Zwischenisolierung oder ohne Zwischenisolierung
mit Aminen der allgemeinen Formel (IV) 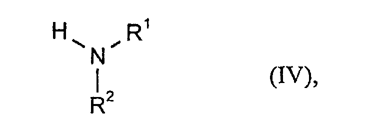 in welcher
R¹ und R² die oben angegebene Bedeutung haben,
in Gegenwart eines Verdünnungsmittels und gegebenenfalls in Gegenwart einer weiteren basischen Verbindung bei Temperaturen zwischen -20°C und +100°C umsetzt ("zweite Verfahrensstufe").

2. Verfahren nach Anspruch 1, **dadurch gekennzeichnet, daß** man die erste Stufe des Verfahrens bei Temperaturen zwischen 50°C und 120°C durchführt.

3. Verfahren nach Anspruch 1, **dadurch gekennzeichnet, daß** man die zweite Stufe des Verfahrens bei Temperaturen zwischen 0°C und 80°C durchführt.

4. Verbindungen der Formel (III) 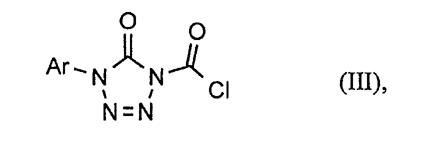 in welcher
Ar für durch Carboxy, Cyano, Carbamoyl, Nitro, Amino, Hydroxy, Halogen, durch C₁-C₄-Alkyl, C₁-C₄-Alkoxy, C₁-C₄-Alkylthio, C₁-C₄-Alkylsulfinyl, C₁-C₄-Alkylsulfunyl, Di-(C₁-C₄-alkyl)amino, C₁-C₄-Alkylsulfonylamino, Di-(C₁-C₄-alkyl)aminosulfonyl, C₁-C₄-Alkylcarbonyl, C₁-C₄-Alkyl-carbonylamino, C₁-C₄-Alkoxy-carbonyl, Di-(C₁-C₄-alkylamino)-carbonyl, C₁-C₄-Alkylendioxy, Phenyl oder Phenoxy (welche jeweils gegebenenfalls durch Fluor und/oder Chlor substituiert sind) substituiertes Phenyl oder Naphthyl steht.

## Claims

1. Process for preparing 1-aryl-4-carbamoyltetrazolinones of the general formula (I) 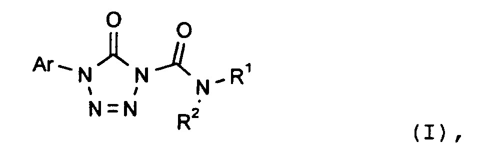 in which
Ar represents phenyl or naphthyl which are optionally substituted by carboxyl, cyano, carbamoyl, nitro, amino, hydroxyl or halogen, or by C₁-C₄-alkyl, C₁-C₄-alkoxy, C₁-C₄-alkylthio, C₁-C₄-alkylsulfinyl, C₁-C₄-alkylsulfonyl, di-(C₁-C₄-alkyl)amino, C₁-C₄-alkylsulfonylamino, di-(C₁-C₄-alkyl)aminosulfonyl, C₁-C₄-alkyl-carbonyl, C₁-C₄-alkyl-carbonylamino, C₁-C₄ -alkoxy-carbonyl, di-(C₁-C₄-alkylamino)carbonyl, C₁-C₄-alkylenedioxy, phenyl or phenoxy (which are in each case optionally substituted by fluorine and/or chlorine),
R¹ represents alkyl, alkenyl or alkinyl which in each case have 1 to 6 carbon atoms and which are in each case optionally substituted by cyano or halogen, and
R² represents alkyl which has 1 to 6 carbon atoms and which is optionally substituted by cyano or halogen, represents alkenyl or alkinyl which have in each case 2 to 6 carbon atoms and which are in each case optionally substituted by cyano or halogen, represents cycloalkyl or cycloalkylalkyl which have in each case 3 to 6 carbon atoms in the cycloalkyl moiety and optionally 1 to 2 carbon atoms in the alkyl moiety and which are in each case optionally substituted by cyano, halogen or C₁-C₄-alkyl, or represents phenyl or phenyl-C₁-C₂-alkyl which are in each case optionally substituted by cyano, nitro or halogen, or by C₁-C₄-alkyl, C₁-C₄-alkoxy, C₁-C₄-alkylthio, C₁-C₄-alkylsulfinyl, C₁-C₄-alkylsulfonyl, di-(C₁-C₄-alkyl)amino, C₁-C₄-alkylsulfonylamino, di-(C₁-C₄-alkyl)aminosulfonyl, C₁-C₄-alkyl-carbonyl or C₁-C₄-alkoxy-carbonyl (which are in each case optionally substituted by fluorine and/or chlorine),
or, together with R¹, represents alkanediyl having 2 to 6 carbon atoms,
**characterized in that** 1-aryl-tetrazolinones of the general formula (II) 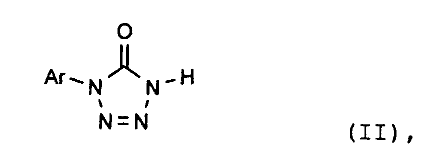 in which
Ar has the abovementioned meaning,
are reacted with phosgene in the presence of a diluent and in the absence of an auxiliary base at temperatures of between 0°C and 150°C ("first process step") and the resulting 1-aryl-4-chlorocarbonyl-tetrazolinones of the general formula (III) 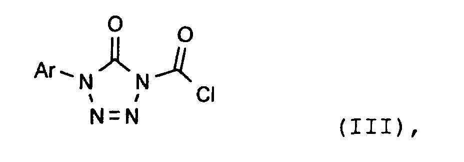 in which
Ar has the abovementioned meaning,
- where appropriate after intermediate isolation or without intermediate isolation -
are reacted with amines of the general formula (IV) 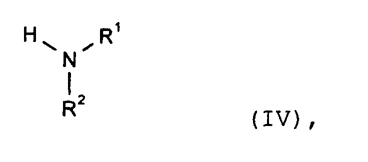 in which
R¹ and R² have the above mentioned meaning,
in the presence of a diluent and, where appropriate, in the presence of a further basic compound at temperatures of between -20°C and +100°C ("second process step").

2. Process according to Claim 1, **characterized in that**, the first step of the process is carried out at temperatures of between 50°C and 120°C.

3. Process according to Claim 1, **characterized in that**, the second step of the process is carried out at temperatures of between 0°C and 80°C.

4. Compounds of the formula (III) 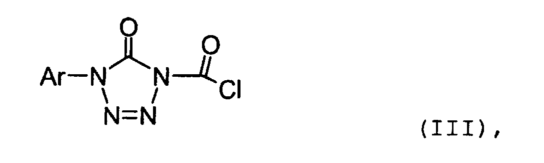 in which
Ar represents phenyl or naphthyl which are substituted by carboxyl, cyano, carbamoyl, nitro, amino, hydroxyl or halogen, or by C₁-C₄-alkyl, C₁-C₄-alkoxy, C₁-C₄-alkylthio, C₁-C₄-alkylsulfinyl, C₁-C₄-alkylsulfonyl, di-(C₁-C₄-alkyl)amino, C₁-C₄-alkylsulfonylamino, di-(C₁-C₄-alkyl)aminosulfonyl, C₁-C₄-alkyl-carbonyl, C₁-C₄-alkyl-carbonylamino, C₁-C₄ -alkoxy-carbonyl, di-(C₁-C₄-alkylamino)-carbonyl, C₁-C₄-alkylenedioxy, phenyl or phenoxy (which are in each case optionally substituted by fluorine and/or chlorine).

## Revendications

1. Procédé pour la préparation de 1-aryl-4-carbamoyl-tétrazolinones répondant à la formule générale (I) 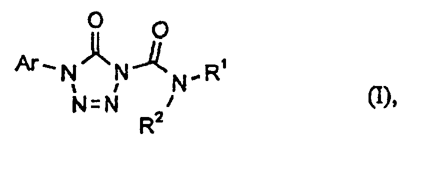 dans laquelle
Ar représente un groupe phényle ou un groupe naphtyle portant, le cas échéant, un ou plusieurs substituants identiques ou différents carboxyle, cyano, carbamoyle, nitro, amino, hydroxyle, halogéno ; le cas échéant, un ou plusieurs substituants identiques ou différents alkyle en C₁-C₄, alcoxy en C₁-C₄, alkyl(en C₁-C₄)thio, alkyl(en C₁-C₄)sulfinyle, alkyl(en C₁-C₄)-sulfonyle, di(alkyl en C₁-C₄)amino, alkyl(en C₁-C₄)sulfonylamino, di(alkyl en C₁-C₄)aminosulfonyle, alkyl(en C₁-C₄)carbonyle, alkyl(en C₁-C₄)carbonylamino, alcoxy(en C₁-C₄)-carbonyle, di(alkyl(en C₁-C₄)amine)carbonyle, alkylène(en C₁-C₄)dioxy, phényle ou phénoxy (chacun de ces substituants portant le cas échéant un ou plusieurs substituants fluoro et/ou chloro),
R¹ représente un groupe alkyle, un groupe alcényle ou un groupe alcynyle contenant respectivement de 1 à 6 atomes de carbone, chacun de ces groupes portant, le cas échéant, un ou plusieurs substituants identiques ou différents cyano ou halogéno,
R² représente un groupe alkyle contenant de 1 à 6 atomes de carbone, portant le cas échéant un ou plusieurs substituants identiques ou différents cyano ou halogéno ; un groupe alcényle ou un groupe alcynyle contenant respectivement de 2 à 6 atomes de carbone, chacun de ces groupes portant le cas échéant un ou plusieurs substituants identiques ou différents cyano ou halogéno ; un groupe cycloalkyle ou un groupe cycloalkylalkyle contenant respectivement de 3 à 6 atomes de carbone dans la fraction cycloalkyle et le cas échéant de 1 à 2 atomes de carbone dans la fraction alkyle, chacun de ces groupes portant le cas échéant un ou plusieurs substituants identiques ou différents cyano, halogéno ou alkyle en C₁-C₄ ; ou représente un groupe phényle ou un groupe phénylalkyle en C₁-C₂, chacun de ces groupes portant, le cas échéant, un ou plusieurs substituants identiques ou différents cyano, nitro, halogéno ; le cas échéant, un ou plusieurs substituants identiques ou différents alkyle en C₁-C₄, alcoxy en C₁-C₄, alkyl(en C₁-C₄)thio, alkyl(en C₁-C₄)sulfinyle, alkyl(en C₁-C₄)-sulfonyle, dialkyl(en C₁-C₄)amino, alkyl(en C₁-C₄)sulfonylamino, dialkyl(en C₁-C₄)aminosulfonyle, alkyl(en C₁-C₄)carbonyle ou alcoxy(en C₁-C₄)carbonyle, (chacun de ces substituants portant le cas échéant
respectivement un ou plusieurs substituants fluoro et/ou chloro),
ou représente, de manière conjointe avec R¹, un groupe alcanediyle contenant de 2 à 6 atomes de carbone,
**caractérisé en ce qu'**on fait réagir des 1-aryl-tétrazolinones répondant à la forme générale (II) 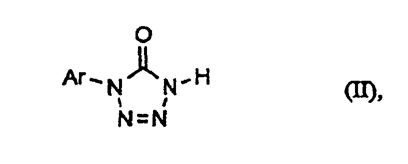 dans laquelle
Ar a la signification indiquée ci-dessus
avec du phosgène en présence d'un diluant et en l'absence d'une base auxiliaire, à des températures entre 0 °C et 150 °C ("première étape opératoire") et on fait réagir les 1-aryl-4-chlorocarbonyle-tétrazolinones obtenues en l'occurrence, répondant à la forme générale (III) 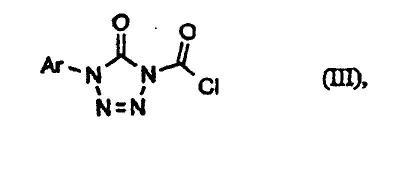 dans laquelle
Ar a la signification indiquée ci-dessus
- le cas échéant après une isolation intermédiaire ou en l'absence d'une isolation intermédiaire -
avec des aminés répondant à la formule générale (IV) 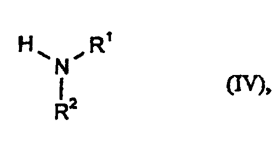 dans laquelle
R¹ et R² ont la signification indiquée ci-dessus,
en présence d'un diluant et, le cas échéant, en présence d'un autre composé basique à des températures entre -20 °C et +100 °C ("deuxième étape opératoire").

2. Procédé selon la revendication 1, **caractérisé en ce qu'**on effectue la première étape du procédé à des températures entre 50 °C et 120 °C.

3. Procédé selon la revendication 1, **caractérisé en ce qu'**on effectue la deuxième étape du procédé à des températures entre 0 °C et 80 °C.

4. Composés répondant à la formule (III) 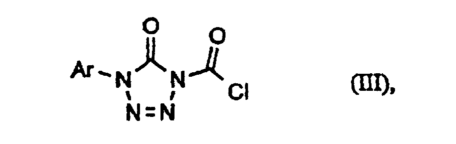 dans laquelle
Ar représente un groupe phényle ou un groupe naphtyle portant, le cas échéant, un ou plusieurs substituants identiques ou différents carboxyle, cyano, carbamoyle, nitro, amino, hydroxyle, halogéno ; le cas échéant, un ou plusieurs substituants identiques ou différents alkyle en C₁-C₄, alcoxy en C₁-C₄, alkyl(en C₁-C₄)thio, alkyl(en C₁-C₄)sulfinyle, alkyl(en C₁-C₄)-sulfonyle, di(alkyl en C₁-C₄)amino, alkyl(en C₁-C₄)sulfonylamino, di(alkyl en C₁-C₄)aminosulfonyle, alkyl(en C₁-C₄)carbonyle, alkyl(en C₁-C₄)carbonylamino, alcoxy(en C₁-C₄)carbonyle, di(alkyl(en C₁-C₄)amino)carbonyle, alkylène(en C₁-C₄)dioxy, phényle ou phénoxy (chacun de ces substituants portant le cas échéant un ou plusieurs substituants fluoro et/ou chloro).
